# EUROPEAN PATENT SPECIFICATION

(11) **EP 1 608 416 B1**
(45) Date of publication and mention of the grant of the patent: **07.01.2009**
(21) Application number: 04758998.1
(22) Date of filing: 26.03.2004
(51) Int. Cl.: A61L 29/02, A61L 31/02, A61L 31/12, A61F 2/06, C22C 14/00, C22C 19/00

(54) **MEDICAL DEVICES HAVING DRUG ELUTING PROPERTIES AND METHODS OF MANUFACTURE THEREOF**
MEDIZINPRODUKTE MIT ARZNEIMITTEL-ELUTIONS-EIGENSCHAFTEN UND HERSTELLUNGSVERFAHREN DAFÜR
DISPOSITIFS MEDICAUX POSSEDANT DES PROPRIETES D'ELUTION DE MEDICAMENTS ET PROCEDES DE FABRICATION DE CEUX-CI

(30) Priority: 31.03.2003 US 459392 P
(43) Date of publication of application: 28.12.2005
(73) Proprietor: Memry Corporation, Bethel, CT 06801 (US)
(72) Inventor: WU, Ming, H., Bethel, CT 06801 (US); PONCET, Philippe, Sandy Hook, CT 06482 (US)
(74) Representative: Kihn, Pierre Emile Joseph
(86) International application number: PCT/US2004/009338
(87) International publication number: WO 2004/091680

(56) References cited:
- EP-A- 0 185 452
- EP-A- 0 824 931
- EP-A- 0 873 734
- EP-A- 1 386 624
- WO-A-96/38594
- WO-A-20/04019820
- US-A- 4 770 725
- US-A1- 2002 026 231
- US-A1- 2004 024 445
- US-B1- 6 375 676
- US-B1- 6 428 634

## Description

### BACKGROUND

The present disclosure relates to medical devices having drug eluting properties and methods of manufacture thereof.

Vascular diseases caused by the progressive blockage of the blood vessels often leads to hypertension, ischemic injury, stroke, or myocardial infarction. Atherosclerotic lesions, which limit or obstruct blood flow, are the major cause of vascular disease. Balloon angioplasty is a medical procedure whose purpose is to increase blood flow through an artery and it is used as a predominant treatment for vessel stenosis. The increasing use of this procedure is attributable to its relatively high success rate and its minimal invasiveness compared with coronary bypass or vascular surgery. A limitation associated with balloon angioplasty is the abrupt or progressive post-procedural re-closure of the vessel or restenosis.

The difficulties associated with balloon angioplasty have facilitated the use of medical devices such as stents and stent technology in most coronary or vascular interventions. The use of such medical devices has significantly reduced the restenosis rate from about 40% after balloon angioplasty alone, to about less than 15% when balloon angioplasty is followed by a subsequent placement of a medical device such as a stent. While contractive remodeling of the vessel is the primary mechanism that leads to restenosis after balloon angioplasty, the restenosis after stent placement is associated with neointimal hyperplasia, which assumed to be caused by vessel injury during stent placement. The in-stent restenosis process occurs first with platelet accumulation on the stent surface. Smooth muscle begins to migrate to the site of the platelet accumulation and proliferate in response to the inflammation. Extracellular matrix finally deposits on the site during the later stages of the healing process. The platelet accumulation and development of extracellular matrix is detrimental to the functioning of the artery.

To battle restenosis, medical devices such as stents often encapsulate drugs or are coated with drugs in order to inhibit or minimize various stages of undesirable cell activity. The pharmacological characteristics of the drugs proposed as coatings for the attenuation of such undesirable cell activity include but are not limited to anti-inflammation, anti-proliferation, immuno-suppressive and anti-migration properties. Examples of such drugs include SIROLIMUS, EVEROLIMUS, ABT 578, PACLITAXEL, DEXAMETHASONE and MYCOPHENOLIC ACID.

Drug coatings generally comprise biologically active agents and polymers. The biologically active agent may be physically blended or encapsulated into a bio-resorbable polymer, to form a drug coating, which is then used to coat the medical device and allowing drug release(s) at various rates post procedurally. Since the polymers utilized in drug coatings generally have glass transition temperatures around room temperature (i.e., about 23°C) they can be designed and fabricated to have sufficient flexibility at temperatures higher than room temperature. However, when cooled to temperatures below the glass transition temperature they are easily embrittled and suffer permanent damage thus rendering them unusable or ineffective.

Some of the alloys used in the manufacture of self-expanding medical devices such as stents (upon which are applied the drug coatings) can be shape memory alloys having a reverse martensitic transformation start temperature (Aₛ) of about 0°C with an austenite transformation finish temperature (A_{f}) of about 20°C to 30°C. Because of the superelastic properties displayed by these alloys at temperatures greater than or equal to about A_{f}, loading a self-expanding medical device into a delivery system at or near ambient temperature is highly challenging as the device often displays a tendency to recover its expanded shape just like a regular spring. To minimize this spring-like phenomena and to achieve free or enhanced loading characteristics into a delivery system, a self-expanding device is generally first cooled to a temperature below its Aₛ temperature, which is also below the ambient temperature. As stated above, this low temperature deformation of the device promotes embrittlement of the drug coating, which often leads to undesirable ruptures or mechanical degradation in the coating.

### SUMMARY

In one embodiment, a medical device comprises a shape memory alloy having a reverse martensitic transformation start temperature of greater than or equal to about 0°C; and a drug coating comprising a polymeric resin and a biologically active agent.

In another embodiment, the medical device is an implantable stent.

In yet another embodiment, a nickel-titanium alloy composition comprises about 55.5 wt% of nickel based on the total composition of the alloy.

In yet another embodiment, a nickel-titanium-niobium alloy composition comprises about 48 wt% nickel and about 14 wt% niobium based on the total composition of the alloy.

In yet another embodiment, a method of manufacturing a stent comprises cold forming a shape memory alloy from a wire; heat treating the cold formed shape memory alloy at a temperatures greater than that at which a martensitic transformation can occur; and coating the stent with a drug coating comprising a biologically active agent.

In yet another embodiment, a method of manufacturing a stent comprises laser cutting, water jet cutting, electrode discharge machining (EDM), chemically, electrochemically or photo-chemically etching a nickel-titanium alloy having about 55.5 wt% of nickel or a nickel-titanium-niobium alloy having about 48 wt% nickel and about 14 wt% niobium from a tube, wherein the weight percents are based on the total weight of the composition; heat treating the alloy at a temperatures greater than that at which a martensitic transformation can occur; and coating the alloy with a drug coating comprising a biologically active agent.

### BRIEF DESCRIPTION OF THE FIGURES

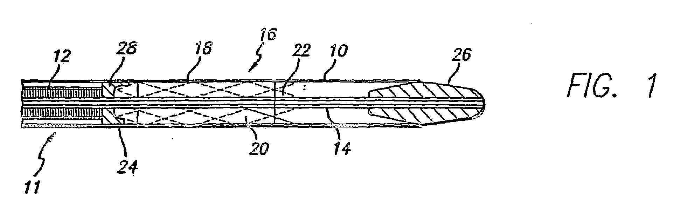

Figure 1 represents a cross-sectional view of the end of a catheter illustrating a stent to be implanted;

Figure 2 is a graphical representation of a tensile stress-strain curve of Ti-55.5 wt% Ni tested at 10°C; and

Figure 3 is a graphical representation of a tensile stress-strain curve of Ti-55.5 wt% Ni tested at 37°C.

### DETAILED DESCRIPTION OF PREFERRED EMBODIMENTS

Disclosed herein is a medical device coated with a drug coating comprising a polymeric resin and a biologically active agent, wherein the medical device is manufactured from an alloy having a reverse martensitic transformation start temperature Aₛ of greater than or equal to about 0°C, preferably greater than or equal to about 10°C and further wherein the polymeric resin also has a glass transition temperature (Tg) of less than or equal to about Aₛ. The use of an alloy having an Aₛ of greater than or equal to about 0°C in conjunction with a drug coating wherein the polymer has a T_{g} is less than or equal to about Aₛ, advantageously allows the medical device to be used at temperatures that are generally lower than sub-ambient temperatures without any permanent deformation and embrittlement of the polymeric resin. Additionally, since the alloy used in the medical device has an Aₛ greater than or equal to about 0°C, the need to cool the medical device to temperatures below 0°C to minimize the "spring-like behavior" is reduced, thereby easing the loading of the device onto the delivery system improving the performance of the medical device post-procedurally.

The medical device may be a stent, a covered stent or stent graft, a needle, a curved needle, bone staples, a vena cava filter, a suture or anchor-like or mechanism. In one exemplary embodiment, the medical device is an implantable stent. A stent as defined herein may be either a solid, hollow, or porous implantable device, which is coated with or encapsulate the drug coating(s). Since the stent may be hollow, solid or porous, the drug coating(s) may be applied to the outer surface, the inner surface, both surfaces of the stent, on selective locations on the stent, for example a different coating could be applied to the ends of a stent compared to its middle portion

The figure illustrates one embodiment of a catheter having an implantable stent. In the figure, the distal end of a catheter 11 having a stent 16 carried within it for implantation into the body of a patient. The proximal end of the catheter 11 is connected to a suitable delivery mechanisms and the catheter 11 is of sufficient length to reach the point of implantation of the stent 16 from the introduction point into the body. The catheter 11 includes an outer sheath 10, a middle tube 12 which may be formed of a compressed spring, and a flexible (e.g., polyamide) inner tube 14. A stent 16 for implantation into a patient is carried within the outer sheath 10. The stent 16 is generally manufactured from a shape memory alloy frame 18, which is formed in a criss-cross pattern, which may be laser cut. One or both ends of the stent 16 may be left uncovered as illustrated at 22 and 24 to provide anchoring within the vessel where the stent 16 is to be implanted.

A radiopaque atraumatic tip 26 is generally secured to the end of the inner tube 14 of the catheter. The atraumatic tip 26 has a rounded end and is gradually sloped to aid in the movement of the catheter through the body vessel. The atraumatic tip 26 is radiopaque so that its location may be monitored by appropriate equipment during the surgical procedure. The inner tube 14 is hollow so as to accommodate a guide wire, which is commonly placed in the vessel prior to insertion of the catheter, although a solid inner section and be used without a guide wire. Inner tube 14 has sufficient kink resistance to engage the vascular anatomy without binding during placement and withdrawal of the delivery system. In addition, inner tube 14 is of sufficient size and strength to allow saline injections without rupture.

A generally cup-shaped element 28 is provided within the catheter 11 adjacent the rear end of the stent 16 and is attached to the end of the spring 12 by appropriate means, e.g., the cup element 28 may be plastic wherein the spring 12 is molded into its base, or the cup element 28 may be stainless steel wherein the spring 12 is secured by welding or the like. The open end of the cup element 28 serves to compress the end 24 of the stent 16 in order to provide a secure interface between the stent 16 and the spring 12. Alternatively, instead of a cup shape, the element 28 could be formed of a simple disk having either a flat or slightly concave surface for contacting the end 24 of the stent 16.

The alloys used in the medical devices are preferably shape memory alloys having an Aₛ greater than or equal to about 0°C. The medical devices may be self expanding or thermally expanding. It is desirable for a self expanding medical device to have the Aₛ of the shape memory alloy be greater than or equal to about 10°C, preferably greater than or equal to about 15°C, preferably greater than or equal to about 20°C, and more preferably greater than or equal to about 23°C. In another embodiment, the shape memory alloys used in the self-expanding medical devices have an A_{f} temperature of about 25°C to about 37°C. Within this range it is generally desirable to have an A_{f} temperature of greater than or equal to about 28°C, preferably greater than or equal to about 30°C. Also desirable within this range is an A_{f} temperature of less than or equal to about 36°C, preferably less than or equal to about 35°C.

If the medical device is thermally expanding, then it is preferable for the shape memory alloys to have an Aₛ greater than or equal to about 35°C. When a medical device is thermally expanding such as is achieved by the use of a hot saline solution, it may be desirable to have an A_{f} temperature of less than or equal to about 50°C.

It is generally desirable to use shape memory alloys having pseudoelastic properties, and which are formable into complex shapes and geometries without the creation of cracks or fractures. It is also generally desirable to use shape memory alloys, which permit large plastic deformations during fabrication of the medical device before the desired pseudoelastic properties are established and wherein the pseudoelastic properties are developed after fabrication.

Shape memory alloys that may be used in the medical devices are generally nickel titanium alloys. Suitable examples of nickel titanium alloys are nickel-titanium-niobium, nickel-titanium-copper, nickel-titanium-iron, nickel-titanium-hafnium, nickel-titanium-palladium, nickel-titanium-gold, nickel-titanium-platinum alloys and the like, and combinations comprising at least one of the foregoing nickel titanium alloys. Preferred alloys are nickel-titanium alloys and titanium-nickel-niobium alloys.

Nickel-titanium alloys that may be used in the medical devices generally comprise nickel in an amount of about 54.5 weight percent (wt%) to about 57.0 wt% based on the total composition of the alloy. Within this range it is generally desirable to use an amount of nickel greater than or equal to about 54.8, preferably greater than or equal to about 55, and more preferably greater than or equal to about 55.1 weight % based on the total composition of the alloy. Also desirable within this range is an amount of nickel less than or equal to about 56.9, preferably less than or equal to about 56.5, and more preferably less than or equal to about 56.0 wt%, based on the total composition of the alloy.

An exemplary composition of a nickel-titanium alloy having an Aₛ greater than or equal to about 0°C is one which comprises about 55.5 wt% nickel (hereinafter Ti-55.5wt%-Ni alloy) based on the total composition of the alloy. The Ti-55.5wt%-Ni alloy has an Aₛ temperature in the fully annealed state of about 30°C. After cold fabrication and shape-setting heat treatment, the Ti-55.5wt%-Ni alloy has an Aₛ of about 10 to about 15°C and an austenite transformation finish temperature (A_{f}) of about 30 to about 35°C.

Another exemplary composition of a nickel-titanium alloy having an Aₛ greater than or equal to about 0°C is one which comprises about 55.8 wt% nickel (hereinafter Ti-55.8wt%-Ni alloy) based on the total composition of the alloy. The Ti-55.8wt%-Ni alloy generally has an Aₛ of 0°C in its as-fabricated state, and an A_{f} of about 15 to about 20°C. However, upon subjecting the Ti-55.8wt%-Ni alloy to aging through annealing, the Aₛ and A_{f} are both increased. The Ti-55.8wt%-Ni alloy has an Aₛ temperature in the fully annealed state of about -10°C. After cold fabrication and shape-setting heat treatment, the Ti-55.8wt%-Ni alloy has an Aₛ of about 0°C and an austenite transformation finish temperature (A_{f}) of about 20°C.

Nickel-titanium-niobium (NiTiNb) alloys that may be used in the medical devices generally comprise nickel in an amount of about 30 wt percent (wt%) to about 56 wt% and niobium in an amount of about 4 wt% to about 43 wt%, with the remainder being titanium. The weight percents are based on the total composition of the alloy. Within the range for nickel, it is generally desirable to use an amount greater than or equal to about 35, preferably greater than or equal to about 40, and more preferably greater than or equal to about 47 wt%, based on the total composition of the alloy. Also desirable within this range is an amount of nickel less than or equal to about 55, preferably less than or equal to about 50, and more preferably less than or equal to about 49 wt%, based on the total composition of the alloy. Within the range for niobium, it is generally desirable to use an amount greater than or equal to about 11, preferably greater than or equal to about 12, and more preferably greater than or equal to about 13 wt%, based on the total composition of the alloy. Also desirable within this range is an amount of niobium less than or equal to about 25, preferably less than or equal to about 20, and more preferably less than or equal to about 16 wt%, based on the total composition of the alloy.

An exemplary composition of a titanium-nickel-niobium alloy is one having about 48 wt% nickel and about 14 wt% niobium, based on the total composition of the alloy. The alloy in the fully annealed state has an Aₛ temperature below the body temperature. However, when subsequently deformed with a properly controlled amount of deformation at a cryogenic temperature, the Aₛ temperature can be elevated above the ambient temperature. The cryogenic temperature as defined herein are temperatures from about -10°C to about -90°C. A NiTiNb alloy can therefore be fabricated in its expanded geometry, annealed and then subsequently deformed to manipulate the Aₛ temperature above the ambient.

The medical devices may be manufactured from the shape memory alloys by a variety of different methods. For example, a medical device such as a stent can be fabricated from wires via cold forming and shape-setting heat treatment process or via warm forming at temperatures above the temperature where martensitic transformation can no longer be mechanically induced. The stent can also be fabricated from nickel-titanium tubes by laser cutting, chemical etching or other cutting means followed by shape-setting heat treatment or other forming and heat treating processes. Once the Aₛ temperature of the stent is above the ambient temperature, the stent may be coated with the drug coating and then crimped into the delivery system at the ambient temperature. During stent deployment, if the A_{f} temperature remains below the body temperature, the stent can be self-expanding and deployed by simply removing the sheath. However, if the A_{f} temperature is above the body temperature, the stent needs to be thermally deployed by, for example, flushing hot saline inside an expansion balloon.

The drug coating used to coat the stent may comprise any polymeric resin having a glass transition temperature less than or equal to about the Aₛ. It is generally desirable for the polymeric resin to have a glass transition temperature greater than or equal to about -100°C, preferably greater than or equal to about-50°C, more preferably greater than or equal to about 0°C, and even more preferably around about 10°C, depending upon the Aₛ of the shape memory alloy utilized in the medical device. In general, the polymeric resin may be derived from a suitable oligomer, polymer, block copolymer, graft copolymer, star block copolymer, dendrimers, ionomers having a number average molecular weight (Mₙ) of about 1000 grams per mole (g/mole) to about 1,000,000 g/mole. The polymeric resin may be either a thermoplastic resin, thermosetting resin or a blend of a thermoplastic resin with a thermosetting resin. Suitable examples of thermoplastic resins include polyacetal, polyacrylic, styrene acrylonitrile, acrylonitrile-butadiene-styrene, polycarbonates, polystyrenes, polyethylene, polypropylenes, polyethylene terephthalate, polybutylene terephthalate, polyamides such as nylon 6, nylon 6,6, nylon 6,10, nylon 6,12, nylon 11 or nylon 12, polyamideimides, polybenzimidazoles, polybenzoxazoles, polybenzothiazoles, polyoxadiazoles, polythiazoles, polyquinoxalines, polyimidazopyrrolones, polyarylates, polyurethanes, thermoplastic olefins such as ethylene propylene diene monomer, ethylene propylene rubber, polyarylsulfone, polyethersulfone, polyphenylene sulfide, polyvinyl chloride, polysulfone, polyetherimide, polytetrafluoroethylene, fluorinated ethylene propylene, perfluoroalkoxy polymer, polychlorotrifluoroethylene, polyvinylidene fluoride, polyvinyl fluoride, polyetherketone, polyether etherketone, polyether ketone ketone, or the like, or combinations comprising at least one of the foregoing thermoplastic resins.

Suitable examples of blends of thermoplastic resins include acrylonitrile-butadiene-styrene/nylon, polycarbonate/acrylonitrile-butadiene-styrene, acrylonitrile butadiene styrene/polyvinyl chloride, polyphenylene ether/polystyrene, polyphenylene ether/nylon, polysulfone/acrylonitrile-butadiene-styrene, polycarbonate/thermoplastic urethane, polycarbonate/polyethylene terephthalate, polycarbonate/polybutylene terephthalate, thermoplastic elastomer alloys, nylon/elastomers, polyester/elastomers, polyethylene terephthalate/polybutylene terephthalate, acetal/elastomer, styrene-maleicanhydride/acrylonitrile-butadiene-styrene, polyether etherketone/polyethersulfone, polyethylene/nylon, polyethylene/polyacetal, or the like, or combinations comprising at least one of the foregoing thermoplastic blends. Suitable examples of polymeric thermosetting materials include polyurethanes, natural rubber, synthetic rubber, epoxy, phenolic, polyesters, polyamides, silicones, or the like, or combinations comprising at least one of the foregoing.

The polymeric resin is generally blended with or encapsulates a biologically active agent to form the drug coating, which is used to coat the medical device. The biologically active agent may also be disposed between layers of polymer to form the drug coating. The biologically active agent is then gradually released from the drug coating, which simply acts as a carrier. When the polymeric resin is physically blended (i.e., not covalently bonded) with the biologically active agent, the release of the biologically active agent from the drug coating is diffusion controlled. It is generally desirable for the drug coating to comprise an amount of about 5 wt% to about 90 wt% of the biologically active agent based on the total weight of the drug coating. Within this range, it is generally desirable to have the biologically active agent present in an amount of greater than or equal to about 10, preferably greater than or equal to about 20, and more preferably greater than or equal to about 30 wt% based on the total weight of the drug coating. Within this range it is generally desirable to have the biologically active agent present in an amount of less than or equal to about 75, preferably less than or equal to about 70, and more preferably less than or equal to about 65 wt% based on the total weight of the drug coating. The drug coating may be optionally coated with an additional surface coating if desired. When an additional surface coating is used, the release of the biologically active agent is interfacially controlled.

In another exemplary embodiment, the biologically active agent may be covalently bonded with a biodegradable polymer to form the drug coating. The rate of release is then controlled by the rate of degradation of the biodegradable polymer. Suitable examples of biodegradable polymers are as polylactic-glycolic acid (PLGA), poly-caprolactone (PCL), copolymers of polylactic-glycolic acid and poly-caprolactone (PCL-PLGA copolymer), polyhydroxy-butyrate-valerate (PHBV), polyorthoester (POE), polyethylene oxide-butylene terephthalate (PEO-PBTP), poly-D,L-lactic acid-*p*-dioxanone-polyethylene glycol block copolymer (PLA-DX-PEG), or the like, or combinations comprising at least one of the foregoing biodegradable polymers.

When the drug coating comprises a biodegradable polymer, it is generally desirable for the biologically active agent to be present in an amount of about 5 wt% to about 90 wt% based on the total weight of the drug coating. Within this range, it is generally desirable to have the biologically active agent present in an amount of greater than or equal to about 10, preferably greater than or equal to about 20, and more preferably greater than or equal to about 30 wt% based on the total weight of the drug coating. Within this range, it is also generally desirable to have the biologically active agent present in an amount of less than or equal to about 75, preferably less than or equal to about 70, and more preferably less than or equal to about 65 wt% based on the total weight of the drug coating.

The drug coating may be coated onto the medical device in a variety of ways. In one embodiment, the drug coating may be dissolved in a solvent such as water, acetone, alcohols such ethanol, isopropanol, methanol, toluene, dimethylformamide, dimethylacetamide and hexane, and coated onto the medical device. In another embodiment, a monomer may be covalently bonded with the biologically active agent and then polymerized to form the drug coating, which is then applied onto the medical device. In yet another embodiment, the polymeric resin may first be applied as a coating onto the medical device, following which the coated device is immersed into the biologically active agent, thus permitting diffusion into the coating to form the drug coating.

It may also be desirable to have two or more biologically active agents dispersed in a single drug coating layer. Alternatively, it may be desirable to have two or more layers of the drug coating coated upon the medical device. Various methods of coating may be employed to coat the medical device such as spin coating, electrostatic painting, dip-coating, plasma or vacuum deposition, painting with a brush, and the like, and combinations comprising at least one of the foregoing methods of coating.

Various types of biologically active agents may be used in the drug coating, which is used to coat the medical device. The coatings on the medical device may be used to deliver therapeutic and pharmaceutic biologically active agents including anti-proliferative/antimitotic agents including natural products such as vinca alkaloids (e.g., vinblastine, vincristine, and vinorelbine), paclitaxel, epidipodophyllotoxins (e.g., etoposide, teniposide), antibiotics (e.g., dactinomycin, actinomycin D, daunorubicin, doxorubicin and idarubicin), anthracyclines, mitoxantrone, bleomycins, plicamycin, mithramycin and mitomycin, enzymes (L-asparaginase, which systemically metabolizes L-asparagine and deprives cells which do not have the capacity to synthesize their own asparagine), antiplatelet agents such as G(GP) IIb/IIIa inhibitors and vitronectin receptor antagonists, antiproliferative/antimitotic alkylating agents such as nitrogen mustards (e.g., mechlorethamine, cyclophosphamide and analogs, melphalan, chlorambucil), ethylenimines and methylmelamines (e.g., hexamethylmelamine and thiotepa), alkyl sulfonates- busulfan, nitrosoureas (e.g., carmustine (BCNU) and analogs, streptozocin), trazenes--dacarbazinine (DTIC), anti-proliferative/antimitotic antimetabolites such as folic acid analogs (e.g., methotrexate), pyrimidine analogs (e.g., fluorouracil, floxuridine, cytarabine), purine analogs and related inhibitors (e.g., mercaptopurine, thioguanine, pentostatin and 2- chlorodeoxyadenosine {cladribine}), platinum coordination complexes (e.g., cisplatin, carboplatin), procarbazine, hydroxyurea, mitotane, aminoglutethimide, hormones (e.g., estrogen), anti-coagulants (e.g., heparin, synthetic heparin salts and other inhibitors of thrombin), fibrinolytic agents (e.g., tissue plasminogen activator, streptokinase and urokinase), aspirin, dipyridamole, ticlopidine, clopidogrel, abciximab, antimigratory, antisecretory (e.g., breveldin), anti-inflammatory: such as adrenocortical steroids (e.g., cortisol, cortisone, fludrocortisone, prednisone, prednisolone, 6α-methylprednisolone, triamcinolone, betamethasone, and dexamethasone), non-steroidal agents (e.g., salicylic acid derivatives such as aspirin, para-aminophenol derivatives such as acetominophen, indole and indene acetic acids (e.g., indomethacin, sulindac, etodalac), heteroaryl acetic acids (e.g., tolmetin, diclofenac, ketorolac), arylpropionic acids (e.g., ibuprofen and derivatives), anthranilic acids (e.g., mefenamic acid, meclofenamic acid), enolic acids (e.g., piroxicam, tenoxicam, phenylbutazone, oxyphenthatrazone), nabumetone, gold compounds (e.g., auranofin, aurothioglucose, gold sodium thiomalate), immunosuppressives (e.g., cyclosporine, tacrolimus (FK-506), sirolimus (e.g., rapamycin, azathioprine, mycophenolate mofetil), angiogenic agents such as vascular endothelial growth factor (VEGF), fibroblast growth factor (FGF), angiotensin receptor blockers, nitric oxide donors, anti-sense oligionucleotides and combinations thereof, cell cycle inhibitors, mTOR inhibitors, and growth factor receptor signal transduction kinase inhibitors, retenoids, cyclin/CDK inhibitors, HMG co-enzyme reductase inhibitors (statins), protease inhibitors.

In one embodiment, a preferred medical device manufactured from an alloy having a reverse martensitic transformation temperature Aₛ greater than or equal to about 10°C, and coated with the drug coating is a stent. Referring now to the figure, in order to deploy the stent 16 inside a body vessel during a surgical procedure, the catheter 11 is introduced into the designated vessel via an introducer positioned at the skin of the patient. A guide wire may have previously been introduced into the vessel, in which case the catheter 11 is introduced by passing the tip 26 over the end of the guide wire outside of the patient and moving the catheter 11 along the path within the vessel, which has been established by the guide wire.

The position of the catheter 11 is tracked by monitoring the tip 26 by means of a fluoroscope. When the catheter 11 is at the desired location i.e., when the stent 16 is positioned at the location where it is be implanted, the movement of the catheter 11 is halted. The catheter 11 must then be removed, leaving the stent 16 in place at the desired location within the vessel. This is accomplished by initially retracting the outer sheath 10, i.e., towards the left in the figure, until it no longer covers the stent 16. The spring 12 is maintained in a fixed position and in conjunction with the cup element 28, serves to maintain the stent 16 in its desired position during the retraction of the outer sheath 10. After the outer sheath 10 has been retracted such that it no longer covers the stent 16 and the stent 16 is expanded, the tip 26 can be pulled back through the stent 16 until the tip 26 abuts the outer sheath 10. As illustrated, the diameter of the tip 26 is slightly greater than the inner diameter of stent 16 when it is inside the outer sheath 10. The stent 16 will expand as it heats up to body temperature as a result of its memory retention characteristics. The tip 26 is then pulled through the center of the stent 16 after the stent 16 has expanded following withdrawal of the sheath 10. Once the tip 26 has been pulled back against the outer sheath 10, the catheter 11 can be removed from the vessel of the patient. This retraction procedure ensures that the tip 26 does not get caught on or embedded in any body vessel when being pulled out of the patient.

The tube spring 12 is maintained stationary during the withdrawal of the outer sheath 10 and serves to keep the stent 16 in its desired location. The tube spring 12 is very well suited for this task since it has extremely low compression in a longitudinal direction once it is fully compressed. It is also well suited for the introduction of the catheter 11 into the body vessel, since it is extremely flexible. Alternatively, other materials, such as various plastics materials, could be employed as the middle tube 12, so long as the compression is low to maintain stent positioning and the necessary flexibility is provided for moving through the vessel. In order to properly deploy the stent 16, the outer sheath 10 must be smoothly retracted while the tube spring 12 maintains its position.

Medical devices made from shape memory alloys having a reverse martensitic transformation temperature Aₛ greater than or equal to about 0°C offer numerous advantages over devices made from alloys having lower Aₛ temperatures. Because of the elevated Aₛ and A_{f} temperatures of the alloys used in the manufacture of the medical device, the effectiveness of the biologically active agent is increased and in addition greater control over the release can be maintained. The use of medical devices having higher reverse martensitic transformation temperatures permits the use of superior drug-eluting coatings which can advantageously possess multiple useful properties such as biocompatibility, improved adhesion to stent, a minimum adhesion to the delivery system, sufficient flexibility and integrity during deployment and invivo, as well as good stability against sterilization processes to which it is subjected during shelf life. For example, a self-expanding stent made from a shape memory alloy having a reverse martensitic transformation temperature of greater than or equal to about 10°C, is ideal for drug coating having a glass transition temperature of greater than or equal to about 10°C. The coated stent can thus be deformed and stayed in its deformed configuration at 10°C where the polymer or the polymerized drug coating stays flexible and ductile without inducing cracks, fractures or delamination. The coated stent can also be loaded onto the delivery catheter at the deforming temperature of 10°C without the resistance of shape recovery. Thus, the stent may be free loaded onto the delivery system without the risks of deforming the coating in its brittle state. Free loading also avoids the potential risk of scraping the coating against cover or sheath, which is generally used to constraint a self-expanding stent during storage, transportation and delivery stages.

The following examples, which are meant to be exemplary, not limiting, illustrate the methods of manufacturing for some of the various embodiments of the medical devices prepared from the shape memory alloys described herein.

### EXAMPLE

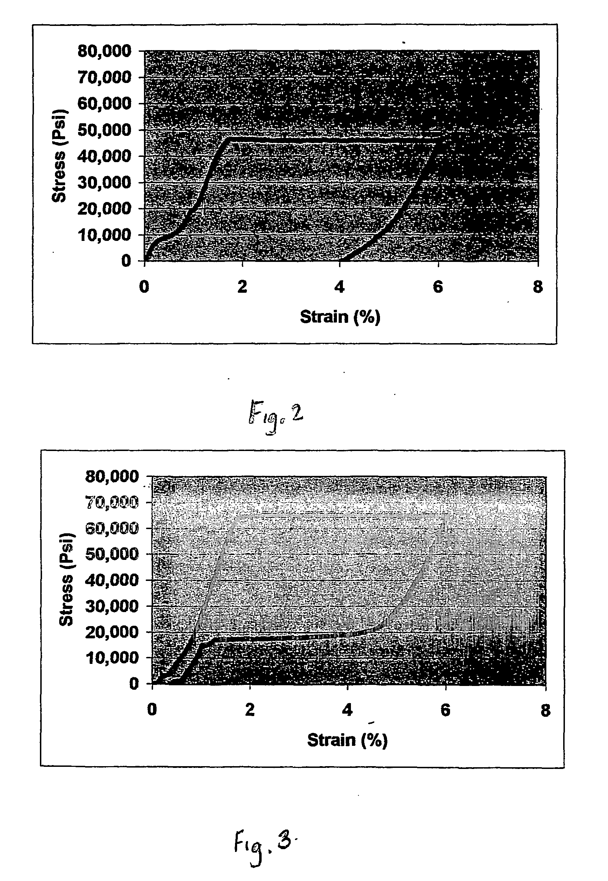

The following example was undertaken to demonstrate the pseudoelastic and superelastic properties of a Ti-55.5wt%-Ni shape memory alloy. The alloy comprises 55.5 wt% nickel with the balance being titanium. The Aₛ of the alloy is 30°C. The alloy was manufactured by vacuum induction melting, followed by secondary vacuum arc re-melting The ingot was hot-forged, hot-rolled and finally cold-drawn to wires of various diameters in the range of about 0.4 to about 5 mm. Inter-pass annealing between cold reductions was carried out at 800°C in an air furnace for wires having a diameter of larger than 2.0 mm or by strand annealing under inert atmosphere for the smaller diameters. Tensile properties were determined using an Instron model 5565 material testing machine equipped with an extensometer of 12.5 mm gage length. The tensile tests were conducted at temperatures of 10°C and at 37°C and the results are shown in Figures 2 and 3 respectively. Figure 2 shows the results of a tensile test conducted at 10°C, from which it may be seen that the alloy upon being subjected to a strain of about 6% recovers only about 2% of the change in length. However at 37°C, the material shows pseudoelastic behavior by returning to its original length.

## Claims

1. A medical device comprising:
a shape memory alloy having a reverse martensitic transformation start temperature of greater than or equal to about 0°C; and
a drug coating comprising a polymeric resin and one or more biologically active agents.

2. The medical device of Claim 1, wherein the shape memory alloy has a reverse martensitic transformation start (Aₛ) temperature of about 10°C to about 15°C.

3. The medical device of Claim 1, wherein the shape memory alloy has a reverse martensitic transformation start (Aₛ) temperature of greater than or equal to about 20°C.

4. The medical device of Claim 1, wherein the shape memory alloy has a transformation finish temperature (A_{f}) of about 25°C to about 50°C.

5. The medical device of Claim 1, wherein the shape memory alloy is a nickel-titanium based alloy.

6. The medical device of Claim 5, wherein the nickel-titanium based alloy is a nickel-titanium-niobium alloy comprising about 30 to 56 wt% nickel, about 4 wt% to about 43 wt% niobium with the remainder being titanium and wherein the weight percents are based on the total composition of the alloy.

7. The medical device of Claim 1, wherein the polymeric resin has a glass transition temperature less than or equal to a reverse martensitic transformation start temperature (Aₛ) of the shape memory alloy.

8. The medical device of Claim 1, wherein the drug coating comprises an amount of about 5 weight percent to about 90 weight percent of the biologically active agent based on the total weight of the drug coating.

9. The medical device of Claim 1, wherein the biologically active agents are copolymerized with the polymeric resin.

10. The medical device of Claim 1, wherein the biologically active agents are dispersed within the polymeric resin.

11. The medical device of Claim 1, wherein the biologically active agents are encapsulated between layers of polymeric resins.

12. The medical device of Claim 1, wherein the polymeric resin is a biodegradable polymer having different biodegradability rates in order to control release drugs at various rates and times or to release multiple drugs with different pharmaceutical behaviors.

13. The medical device of Claim 12, wherein the biodegradable polymer is a polylactic-glycolic acid, poly-caprolactone, copolymer of polylactic-glycolic acid and poly-caprolactone, polyhydroxy-butyrate-valerate, polyorthoester, polyethylene oxide-butylene terephthalate, poly-D,L-lactic acid-*p*-dioxanone-polyethylene glycol block copolymer or a combination comprising at least one of the foregoing biodegradable polymers.

14. The medical device of Claim 1, wherein the device is an implantable device.

15. The medical device of Claim 14, wherein the implantable device is a stent, bone staple, a vena cava filter, a suture or anchor-like mechanism.

16. A method of manufacturing a stent comprising:
cold forming a shape memory alloy from a wire;
heat treating the cold formed, shape memory alloy at a temperatures greater than that at which a martensitic transformation can occur; and
coating the stent with a drug coating comprising a biologically active agent.

17. The method of Claim 16, wherein the shape memory alloy has a reverse martensitic transformation start (Aₛ) temperature of greater than or equal to about 0°C.

18. The method of Claim 16, wherein the shape memory alloy has a transformation finish temperature (A_{f}) of about 25°C to about 50°C.

19. The method of Claim 16, wherein the shape memory alloy is a nickel-titanium based alloy.

20. The method of Claim 16, wherein the drug coating further comprises a polymeric resin having a glass transition temperature greater than or equal to about-180°C and wherein the polymeric resin is a thermoplastic resin, thermosetting resin or a blend of a thermoplastic resin with a thermosetting resin.

21. The method of Claim 20, wherein the polymeric resin is biodegradable.

22. A method of manufacturing a stent comprising:
laser cutting or chemically etching a nickel-titanium alloy having about 55.5 weight percent of nickel or a nickel-titanium-niobium alloy having about 48 weight percent nickel and about 14 weight percent niobium from a tube, wherein the weight percents are based on the total weight of the composition;
heat treating the alloy at a temperatures greater than that at which a martensitic transformation can occur; and
coating the alloy with a drug coating comprising a biologically active agent.

## Patentansprüche

1. Medizinische Vorrichtung, umfassend:
eine Formgedächtnislegierung, die eine Martensitrückumwandlungs-Starttemperatur von größer als oder gleich etwa 0 °C aufweist; und
eine Arzneimittelbeschichtung, die ein Polymerharz und ein oder mehrere biologisch wirksame Mittel umfasst.

2. Medizinische Vorrichtung nach Anspruch 1, wobei die Formgedächtnislegierung eine Martensitrückumwandlungs-Starttemperatur (Aₛ) von etwa 10 °C bis etwa 15 °C aufweist

3. Medizinische Vorrichtung nach Anspruch 1, wobei die Formgedächtnislegierung eine Martensitrückumwandlungs-Starttemperatur (Aₛ) von größer als oder gleich etwa 20 °C aufweist.

4. Medizinische Vorrichtung nach Anspruch 1, wobei die Formgedächtnislegierung eine Umwandlungs-Endtemperatur (A_{f}) von etwa 25 °C bis etwa 50 °C aufweist

5. Medizinische Vorrichtung nach Anspruch 1, wobei die Formgedächtnislegierung eine Legierung auf Nickel-Titan-Basis ist

6. Medizinische Vorrichtung nach Anspruch 5, wobei die Legierung auf Nickel-Titan-Basis eine Nickel-Titan-Niob-Legierung ist, die etwa 30 bis 56 Gew.-% Nickel, etwa 4 Gew.-% bis etwa 43 Gew.-% Niob umfasst, wobei der Rest Titan ist und wobei die Gewichtsprozente auf der Gesamtzusammensetzung der Legierung basieren.

7. Medizinische Vorrichtung nach Anspruch 1, wobei das Polymerharz eine Glasübergangstemperatur aufweist, die kleiner als eine oder gleich einer Martensitrückumwandlungs-Starttemperatur (Aₛ) der Formgedächtnislegierung ist

8. Medizinische Vorrichtung nach Anspruch 1, wobei die Arzneimittelbeschichtung, basierend auf dem Gesamtgewicht der Arzneimittelbeschichtung, eine Menge von etwa 5 Gewichtsprozenten bis etwa 90 Gewichtsprozenten des biologisch wirksamen Mittels umfasst

9. Medizinische Vorrichtung nach Anspruch 1, wobei die biologisch wirksamen Mittel mit dem Polymerharz copolymerisiert sind.

10. Medizinische Vorrichtung nach Anspruch 1, wobei die biologisch wirksamen Mittel in dem Polymerharz dispergiert sind

11. Medizinische Vorrichtung nach Anspruch 1, wobei die biologisch wirksamen Mittel zwischen Schichten aus Polymerharzen eingekapselt sind.

12. Medizinische Vorrichtung nach Anspruch 1, wobei das Polymerharz ein biologisch abbaubares Polymer ist, das verschiedene biologische Abbaubarkeitsraten aufweist, um Arzneimittel mit diversen Raten und zu diversen Zeiten kontrolliert freizugeben oder um mehrere Arzneimittel mit unterschiedlichem pharmazeutischem Verhalten freizugeben.

13. Medizinische Vorrichtung nach Anspruch 12, wobei das biologisch abbaubare Polymer eine Polymilch-Glykolsäure, ein Polycaprolacton, ein Copolymer aus Polymilch-Glykolsäure und Polycaprolacton, ein Polyhydroxybutyrat-valerat, Polyorthoester, Polyethylenoxid-butylenterephthalat, Poly-D,L-Milchsäure-*p*-dioxanon-polyethylen-glykol-Blockcopolymer oder eine Kombination ist, die mindestens eines der vorstehenden biologisch abbaubaren Polymere umfasst

14. Medizinische Vorrichtung nach Anspruch 1, wobei die Vorrichtung eine implantierbare Vorrichtung ist

15. Medizinische Vorrichtung nach Anspruch 14, wobei die implantierbare Vorrichtung ein Stent, eine Knochenklammer, ein Vena-cava-Filter, ein Nahtmaterial oder ankerähnlicher Mechanismus ist

16. Verfahren zur Herstellung eines Stents, umfassend:
Kaltformen einer Formgedächtnislegierung aus einem Draht;
Wärmebehandeln der kaltgeformten Formgedächtnislegierung bei Temperaturen, die größer sind als jene, bei der eine Martensitumwandlung auftreten kann; und
Beschichten des Stents mit einer Arzneimittelbeschichtung, die ein biologisch wirksames Mittel umfasst

17. Verfahren nach Anspruch 16, wobei die Formgedächtnislegierung eine Martensitrückumwandlungs-Starttemperatur (Aₛ) von größer als oder gleich etwa 0 °C aufweist

18. Verfahren nach Anspruch 16, wobei die Formgedächtnislegierung eine Umwandlungs-Endtemperatur (A_{f}) von etwa 25 °C bis etwa 50 °C aufweist.

19. Verfahren nach Anspruch 16, wobei die Formgedächtnislegierung eine Legierung auf Nickel-Titan-Basis ist.

20. Verfahren nach Anspruch 16, wobei die Arzneimittelbeschichtung außerdem ein Polymerharz umfasst, das eine Glasübergangstemperatur von größer als oder gleich etwa -180°C aufweist und wobei das Polymerharz ein thermoplastisches Harz, ein hitzehärtbares Harz oder eine Mischung eines thermoplastischen Harzes mit einem hitzehärtbaren Harz ist

21. Verfahren nach Anspruch 20, wobei das Polymerharz biologisch abbaubar ist

22. Verfahren zur Herstellung eines Stents, umfassend:
Laserschneiden oder chemisches Ätzen einer Nickel-Titan-Legierung mit etwa 55,5 Gewichtsprozenten Nickel oder einer Nickel-Titan-Niob-Legierung mit etwa 48 Gewichtsprozenten Nickel und etwa 14 Gewichtsprozenten Niob aus einem Röhrchen, wobei die Gewichtsprozente auf dem Gesamtgewicht der Zusammensetzung basieren;
Wärmebehandeln der Legierung bei Temperaturen, die größer ist als jene, bei der eine Martensitumwandlung auftreten kann; und
Beschichten der Legierung mit einer Arzneimittelbeschichtung, die ein biologisch wirksames Mittel umfasst.

## Revendications

1. Dispositif médical comprenant:
un alliage à mémoire de forme ayant une température de début de transformation martensitique inverse supérieure ou égale à environ 0°C ; et
un revêtement médicamenteux comprenant une résine polymérique et un ou plusieurs agent(s) biologiquement actif(s)

2. Dispositif médical selon la revendication 1, dans lequel l'alliage à mémoire de forme a une température de début de transformation martensitique inverse (Aₛ) d'environ 10°C à environ 15°C.

3. Dispositif médical selon la revendication 1, dans lequel l'alliage à mémoire de forme a une température de début de transformation martensitique inverse (Aₛ) supérieure ou égale à environ 20°C.

4. Dispositif médical selon la revendication 1, dans lequel l'alliage à mémoire de forme a une température de fin de transformation (A_{f}) d'environ 25°C à environ 50°C.

5. Dispositif médical selon la revendication 1, dans lequel l'alliage à mémoire de forme est un alliage à base de nickel-titane.

6. Dispositif médical selon la revendication 5, dans lequel l'alliage à base de nickel-titane est un alliage de nickel-titane-niobium comprenant environ 30 à 56 % en poids de nickel, environ 4 % en poids à environ 43 % en poids de niobium avec le reste étant du titane et dans lequel les pourcentages en poids sont basés sur la composition totale de l'alliage

7. Dispositif médical selon la revendication 1, dans lequel la résine polymérique a une température de transition vitreuse inférieure ou égale à une température de début de transformation martensitique inverse (Aₛ) de l'alliage à mémoire de forme

8. Dispositif médical selon la revendication 1, dans lequel le revêtement médicamenteux comprend une quantité d'environ 5 pourcents en poids à environ 90 pourcents en poids de l'agent biologiquement actif sur la base du poids total du revêtement médicamenteux.

9. Dispositif médical selon la revendication 1, dans lequel les agents biologiquement actifs sont copolymérisés avec la résine polymérique.

10. Dispositif médical selon la revendication 1, dans lequel les agents biologiquement actifs sont dispersés au sein de la résine polymérique.

11. Dispositif médical selon la revendication 1, dans lequel les agents biologiquement actifs sont encapsulés entre des couches de résines polymériques.

12. Dispositif médical selon la revendication 1, dans lequel la résine polymérique est un polymère biodégradable ayant différentes vitesses de biodégradabilité afin de contrôler la libération des médicaments à diverses vitesses et divers moments ou de libérer de multiples médicaments avec différents comportements pharmaceutiques.

13. Dispositif médical selon la revendication 12, dans lequel le polymère biodégradable est un acide polylactique-glycolique, un polycaprolactone, un copolymère d'acide polylactique-glycolique et de polycaprolactone, un polyhydroxy-butyrate-valérate, un polyorthoester, un oxyde de polyéthylène-téréphtalate de butylène, un copolymère séquencé poly D,L-acide lactique-p-dioxanone-polyéthylèneglycol ou une combinaison comprenant au moins un des polymères biodégradables qui précèdent.

14. Dispositif médical selon la revendication 1, dans lequel le dispositif est un dispositif implantable.

15. Dispositif médical selon la revendication 14, dans lequel le dispositif implantable est un stent, une agrafe osseuse, un filtre pour veine cave, une suture ou un mécanisme de type ancre

16. Procédé de fabrication d'un stent comprenant :
la formation à froid d'un alliage à mémoire de forme à partir d'un fil ;
le traitement à chaud de l'alliage à mémoire de forme, formé à froid à des températures supérieures à celles auxquelles une transformation martensitique peut se produire ; et
l'enrobage du stent avec un revêtement médicamenteux comprenant un agent biologiquement actif

17. Procédé selon la revendication 16, dans lequel l'alliage à mémoire de forme a une température de début de transformation martensitique inverse (Aₛ) supérieure ou égale à environ 0°C.

18. Procédé selon la revendication 16, dans lequel l'alliage à mémoire de forme a une température de fin de transformation (A_{f}) d'environ 25°C à environ 50°C.

19. Procédé selon la revendication 16, dans lequel l'alliage à mémoire de forme est un alliage à base de nickel-titane.

20. Procédé selon la revendication 16, dans lequel le revêtement médicamenteux comprend en outre une résine polymérique ayant une température de transition vitreuse supérieure ou égale à environ -180°C et dans lequel la résine polymérique est une résine thermoplastique, une résine thermodurcissable ou un mélange d'une résine thermoplastique avec une résine thermodurcissable.

21. Procédé selon la revendication 20, dans lequel la résine polymérique est biodégradable

22. Procédé de fabrication d'un stent comprenant :
le découpage au laser ou la gravure par voie chimique d'un alliage de nickel-titane ayant environ 55,5 pourcents en poids de nickel ou d'un alliage de nickel-titane-niobium ayant environ 48 pourcents en poids de nickel et environ 14 pourcents en poids de niobium à partir d'un tube, dans lequel les pourcentages en poids sont basés sur le poids total de la composition ;
le traitement à chaud de l'alliage à des températures supérieures à celles auxquelles une transformation martensitique peut se produire ; et
l'enrobage de l'alliage avec un revêtement médicamenteux comprenant un agent biologiquement actif.
